(19) Europäisches Patentamt
European Patent Office
Office européen des brevets

(11) EP 1 615 896 B1

(12) **EUROPEAN PATENT SPECIFICATION**

(45) Date of publication and mention of the grant of the patent:
**23.04.2008 Bulletin 2008/17**

(21) Application number: **04725384.4**

(22) Date of filing: **02.04.2004**

(51) Int Cl.:
***C07D 235/06*** (2006.01) ***C07D 401/10*** (2006.01)
***A61K 31/4184*** (2006.01) ***A61K 31/454*** (2006.01)
***A61K 31/496*** (2006.01) ***A61P 21/02*** (2006.01)
***A61P 23/00*** (2006.01) ***A61P 25/08*** (2006.01)
***A61P 25/20*** (2006.01) ***A61P 25/22*** (2006.01)

(86) International application number:
**PCT/EP2004/050427**

(87) International publication number:
**WO 2004/089912 (21.10.2004 Gazette 2004/43)**

(54) **NOVEL BENZIMIDAZOLE DERIVATIVES AND PHARMACEUTICAL COMPOSITIONS COMPRISING THESE COMPOUNDS**

NEUE BENZIMIDAZOLDERIVATIVE UND IHRE PHARMAZEUTISCHE ZUSAMMENSETZUNGEN

NOUVEAU DERIVES DE BENZIMIDAZOLE ET COMPOSITIONS PHARMACEUTIQUES COMPRENANT CES COMPOSES

(84) Designated Contracting States:
**AT BE BG CH CY CZ DE DK EE ES FI FR GB GR HU IE IT LI LU MC NL PL PT RO SE SI SK TR**

(30) Priority: **10.04.2003 DK 200300557**
**11.04.2003 US 461794 P**

(43) Date of publication of application:
**18.01.2006 Bulletin 2006/03**

(73) Proprietor: **NEUROSEARCH A/S**
**2750 Ballerup (DK)**

(72) Inventors:
- **LARSEN, Janus S.,**
**c/o NeuroSearch A/S**
**DK-2750 Ballerup (DK)**
- **TEUBER, Lene,**
**c/o NeuroSearch A/S**
**DK-2750 Ballerup (DK)**

(74) Representative: **Abildgren, Michael Padkjaer et al**
**NeuroSearch A/S**
**Patent Department**
**93 Pederstrupvej**
**2750 Ballerup (DK)**

(56) References cited:
**WO-A-00/78728** **WO-A-02/50057**
**WO-A-98/17651**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### TECHNICAL FIELD

The present invention relates to novel benzimidazole derivatives and pharmaceutical compositions containing these compounds.

The compounds of the invention are useful in the treatment of central nervous system diseases and disorders, which are responsive to modulation of the $GABA_A$ receptor complex, and in particular for inducing and maintaining anaesthesia, sedation and muscle relaxation, as well as for combating febrile convulsions in children.

The compounds of the invention may also be used by veterinarians.

### BACKGROUND ART

Agents that bind or interact with the modulatory sites on the $GABA_A$ receptor complex, such as for example the benzodiazepine receptor, can have either enhancing effect on the action of GABA, i.e. a positive modulatory effect of the receptor (agonists, partial agonists), an attenuating effect on the action of GABA, i.e. negative modulation of the receptor (inverse agonists, partial inverse agonists), or they can block the effect of both agonists and inverse agonists (antagonists or ligands without intrinsic activity).

Agonists generally produce muscle relaxant, hypnotic, sedative, anxiolytic, and/or anticonvulsant effects, while inverse agonists produce pro-convulsive, anti-inebriant or anxiogenic effects. Compounds with anxiolytic effects, but with or without reduced muscle relaxant, hypnotic and sedative effects, are characterised as partial agonists. Partial inverse agonists are considered to be useful as cognition enhancers.

Full agonists of the benzodiazepine receptor are considered useful as anaesthetics. However, many drugs presently available as anaesthetics, and especially pre-anaesthetics, give rise to hang-over effects as well as long awakening times, wherein careful monitoring of the patient is necessary. Anaesthetics with a long half-life may also impose difficulties during incidents of overdosing i.e. prolonged respiratory depression. Furthermore, some currently used drugs cannot be used for anaesthetising children as deaths have been reported in children after prolonged use of Propofol. Some anaesthetics are gasses, which inherently possesses a contamination problem for the medical staff.

A well known anaesthetic, Propofol, is administered as a mixture of soybean oil, glycerol and purified egg phosphatide, which mixture nourish bacterial growth. Administration of bacterially contaminated Propofol has been reported to cause sepsis and death [Wiklund et al.; The New England Journal of Medicine 1997 337 (16) 1132-1141]. Further, compounds with a long *in vivo* half-life will give problems with accumulation during and after prolonged treatment e.g. when administered to patients constrained to a respirator. Short half-lives wherein the compounds are metabolised to inactive metabolites allow for a predictable correlation of dose and duration of pharmacological effect.

Ideally the anaesthestising effect should be observed shortly after a bolus injection or infusion of the compound. A rapid onset of action minimises the period of anxiety and uneasiness experienced by patients going into surgery.

Patients suffering from severe and continuous epileptic attacks presently treated with large amounts of sedatives, e.g. benzodiazepines, will benefit from shorter acting compounds with no hang-over or long lasting sedating effect.

As the preferred route of administration is by intravenous injection or infusion, the anaesthestising compounds should preferably be water soluble.

EP 616807 describes benzimidazole compounds for use as benzodiazepine receptor ligands.

WO 96/33194, WO 96/33191 and WO 96/33192 describe benzimidazole compounds having affinity for the GABA receptor complex.

WO 98/34923 describes phenylbenzimidazole derivatives as ligands for the GABA receptor complex.

WO 98/17651, WO 00/78728 and WO 02/050057 describe benzimidazole compounds for use as e.g. anaesthetics.

However, there is a continued strong need to find compounds with an optimized pharmacological profile.

### SUMMARY OF THE INVENTION

It is an object of the invention to provide novel compounds useful as anaesthetics and/or pre-anaesthetics, sedatives, muscle relaxants, and for the treatment of febrile convulsions in children, status epilepticus, for use to patients constrained to a respirator as well as for veterinarian uses. A further object of the invention is to produce compounds which show a rapid onset of action. A still further object of the invention is to produce compounds with less hang-over effect and/or less long lasting sedation effect thereby showing a faster recovery of the patients.

In its first aspect, the invention provides a compound of general formula I:

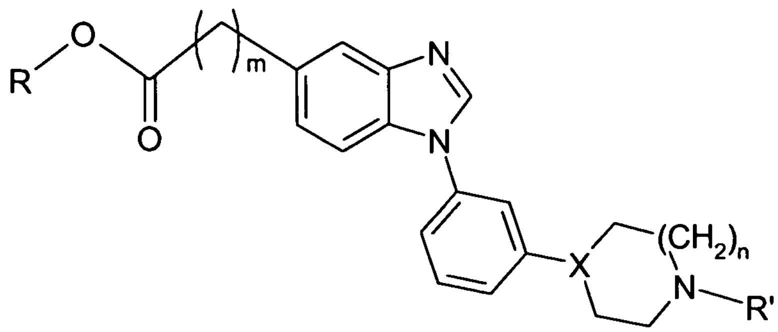

( I )

or an N-oxide thereof, or a pharmaceutically acceptable salt thereof, wherein R, R', X, m and n are defined as below.

In its second aspect, the invention provides a pharmaceutical composition containing a therapeutically effective amount of a compound according to the invention, or an N-oxide thereof, or a pharmaceutically acceptable salt thereof, together with at least one pharmaceutically acceptable carrier, excipient or diluent.

In its third aspect, the invention provides a use of a compound according to the invention, or an N-oxide thereof, or a pharmaceutically acceptable salt thereof, for the manufacture of a medicament for the treatment, prevention or alleviation of a disease or a disorder or a condition of a mammal, including a human, which disease, disorder or condition is responsive to modulation of the GABA receptor complex.

Other objects of the invention will be apparent to the person skilled in the art from the following detailed description and the working examples.

## DETAILED DISCLOSURE OF THE INVENTION

### Benzimidazole Derivatives

In its first aspect the invention provides novel compounds. The compounds of the invention are represented by general formula I:

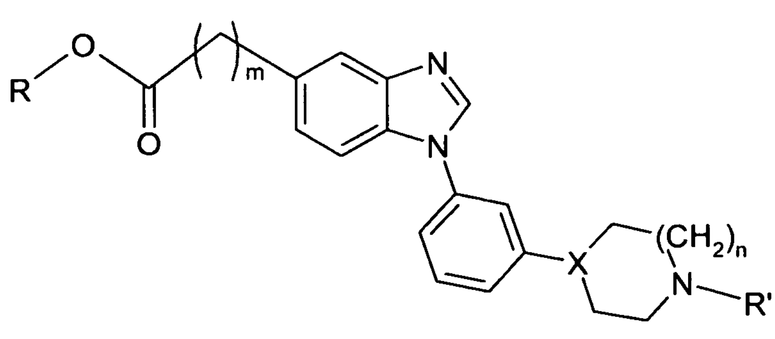

( I )

or an N-oxide thereof, or a pharmaceutically acceptable salt thereof, wherein
R represents $C_{1\text{-}6}$-alkyl, hydroxy-$C_{1\text{-}6}$-alkyl, $C_{1\text{-}6}$-alkoxy-$C_{1\text{-}6}$-alkyl, $R^aR^b$N-$C_{1\text{-}6}$-alkyl, $R^aR^b$N-CO-$C_{1\text{-}6}$-alkyl, or phenyl-$C_{1\text{-}6}$-alkyl;
wherein $R^a$ and $R^b$ independently of each other represents hydrogen or $C_{1\text{-}6}$-alkyl;
R' represents $C_{1\text{-}6}$-alkoxy-$C_{1\text{-}6}$-alkyl, $C_{1\text{-}6}$-alkoxy-$C_{2\text{-}6}$-alkenyl or $C_{1\text{-}6}$-alkoxy-$C_{2\text{-}6}$-alkynyl,;
m is 0 or 1;
n is 1; and
X represents N or CH.

In one embodiment of the compound general formula I, n is 1 and X represents N.

In a second embodiment of the compound of general formula I, n is 1 and X represents CH.

In a further embodiment of the compound of general formula I, R represents $C_{1\text{-}6}$-alkyl, hydroxy-$C_{1\text{-}6}$-alkyl, or $C_{1\text{-}6}$-alkoxy-$C_{1\text{-}6}$-alkyl. In a special embodiment, R represents $C_{1\text{-}6}$-alkyl, such as methyl, ethyl, n-butyl, or iso-butyl. In a further embodiment, R represents hydroxy-$C_{1\text{-}6}$-alkyl, such as hydroxymethyl or hydroxyethyl. In a still further embodiment, R represents $C_{1\text{-}6}$-alkoxy-$C_{1\text{-}6}$-alkyl. In a special embodiment, R represents $C_{1\text{-}6}$-alkoxyethyl, such as methox-

yethyl.

In a further embodiment of the compound of general formula I, R represents $R^aR^bN$-$C_{1-6}$-alkyl, $R^aR^bN$-CO-$C_{1-6}$-alkyl, or phenyl- $C_{1-6}$-alkyl. In a further embodiment, R represents $R^aR^bN$-$C_{1-6}$-alkyl or $R^aR^bN$-CO-$C_{1-6}$-alkyl, wherein $R^a$ and $R^b$ independently of each other represents hydrogen, methyl or ethyl. In a special embodiment, R represents $R^aR^bN$-$C_{1-6}$-alkyl. In a further embodiment, R represents methylamino-$C_{1-6}$-alkyl or dimethylamino-$C_{1-6}$-alkyl, such as methylaminoethyl or dimethylaminoethyl. In a still further embodiment, R represents $R^aR^bN$-CO-$C_{1-6}$-alkyl. In a further embodiment, R represents methylcarbamoyl-$C_{1-6}$-alkyl, ethylcarbamoyl-$C_{1-6}$-alkyl, or dimethylcarbamoyl-$C_{1-6}$-alkyl, such as methylcarbamoylmethyl, ethylcarbamoylmethyl or dimethylcarbamoylmethyl. In a still further embodiment, R represents phenyl-$C_{1-6}$-alkyl, such as phenylmethyl.

In a further embodiment of the compound of general formula I, $R^a$ and $R^b$ independently of each other represents hydrogen, methyl or ethyl. In one embodiment, $R^a$ represents hydrogen. In a second embodiment, $R^a$ represents methyl. In a further embodiment, $R^a$ represents ethyl. In a still further embodiment, $R^b$ represents hydrogen. In a further embodiment, $R^b$ represents methyl. In a still further embodiment, $R^b$ represents ethyl. In a further embodiment, $R^a$ represents methyl and $R^b$ represents methyl. In a still further embodiment, $R^a$ represents methyl and $R^b$ represents hydrogen. In a further embodiment, $R^a$ represents ethyl and $R^b$ represents hydrogen.

In a further embodiment of the compound of general formula I, R' represents $C_1$-$_6$-alkoxy- $C_{1-6}$-alkyl. In a special embodiment, R' represents $C_{1-6}$-alkoxyethyl, such as methoxyethyl or ethoxyethyl. In a further embodiment, R' represents methoxy-$C_{1-6}$-alkyl. In a still further embodiment, R' represents ethoxy-$C_{1-6}$-alkyl.

In a still further embodiment of the compound of general formula I, R represents $C_{1-6}$-alkyl, R' represents $C_{1-6}$-alkoxy-$C_{1-6}$-alkyl, m is 0, n is 1 and X represents N.

In a further embodiment of the compound of general formula I, R represents hydroxy-$C_{1-6}$-alkyl, R' represents $C_{1-6}$-alkoxy-$C_{1-6}$-alkyl, m is 0, n is 1 and X represents N.

In a still further embodiment of the compound of general formula I, R represents $C_{1-6}$-alkoxy-$C_{1-6}$-alkyl, R' represents $C_{1-6}$-alkoxy-$C_{1-6}$-alkyl, m is 0, n is 1 and X represents N.

In a still further embodiment of the compound of general formula I, R represents $C_{1-6}$-alkyl, R' represents $C_{1-6}$-alkoxy-$C_{1-6}$-alkyl, m is 1, n is 1 and X represents N.

In a further embodiment of the compound of general formula I, R represents hydroxy-$C_{1-6}$-alkyl, R' represents $C_{1-6}$-alkoxy-$C_{1-6}$-alkyl, m is 1, n is 1 and X represents N.

In a still further embodiment of the compound of general formula I, R represents $C_{1-6}$-alkyl, R' represents $C_{1-6}$-alkoxy-$C_{1-6}$-alkyl, m is 0, n is 1 and X represents CH.

In a still further embodiment of the compound of general formula I, R represents $C_{1-6}$-alkoxy-$C_{1-6}$-alkyl, R' represents $C_{1-6}$-alkoxy-$C_{1-6}$-alkyl, m is 0, n is 1 and X represents CH.

In a further embodiment of the compound of general formula I, R represents phenyl-$C_{1-6}$-alkyl, R' represents $C_{1-6}$-alkoxy-$C_{1-6}$-alkyl, m is 0, n is 1 and X represents N.

In a still further embodiment of the compound of general formula I, R represents $R^aR^bN$-$C_{1-6}$-alkyl, R' represents $C_{1-6}$-alkoxy-$C_{1-6}$-alkyl, m is 0, n is 1 and X represents N.

In a still further embodiment of the compound of general formula I, R represents $R^aR^bN$-CO-$C_1$-$_6$-alkyl, R' represents $C_{1-6}$-alkoxy-$C_{1-6}$-alkyl, m is 0, n is 1 and X represents N.

In a special embodiment the chemical compound of the invention is

2-Methoxyethyl 1-(3-(4-(ethoxyethyl)-1-piperazinyl)-phenyl)-benzimidazole-5-carboxylate (**1a**);
2-Hydroxyethyl 1-(3-(4-ethoxyethyl)-1-piperazinyl)-phenyl)-benzimidazole-5-carboxylate (**1b**);
*n*-Butyl 1-(3-(4-(ethoxyethyl)-1-piperazinyl)-phenyl)-benzimidazole-5-carboxylate (**1c**);
*iso*-Butyl 1-(3-(4-(ethoxyethyl)-1-piperazinyl)-phenyl)-benzimidazole-5-carboxylate (**1d**);
2-Methoxyethyl 1-(3-(4-(methoxyethyl)-1-piperazinyl)-phenyl)-benzimidazole-5-carboxylate (**1e**);
2-Hydroxyethyl 1-(3-(4-(methoxyethyl)-1-piperazinyl)-phenyl)-benzimidazole-5-carboxylate (**1f**);
*n*-Butyl 1-(3-(4-(methoxyethyl)-1-piperazinyl)-phenyl)-benzimidazole-5-carboxylate (**1g**);
*iso*-Butyl 1-(3-(4-(methoxyethyl)-1-piperazinyl)-phenyl)-benzimidazole-5-carboxylate (**1h**);
5-(methoxycarbonylmethyl) 1-(3-(4-(methoxyethyl)-1-piperazinyl)-phenyl)-benzimidazole (**1i**);
5-(2-hydroxyethoxycarbonylmethyl) 1-(3-(4-(methoxyethyl)-1-piperazinyl)-phenyl)-benzimidazole (**1j**);
2-Methoxyethyl 1-(3-(1-methoxyethyl-4-piperidinyl)-phenyl)-benzimidazole-5-carboxylate (**8d**);
Benzyl 1-(3-(4-(ethoxyethyl)-1-piperazinyl)-phenyl)-benzimidazole-5-carboxylate **(10a);**
Methylcarbamoylmethyl 1-(3-(4-(ethoxyethyl)-1-piperazinyl)-phenyl)-benzimidazole-5-carboxylate (**10b**);
Ethylcarbamoylmethyl 1-(3-(4-(ethoxyethyl)-1-piperazinyl)-phenyl)-benzimidazole-5-carboxylate (**10c**);
2-Dimethylaminoethyl 1-(3-(4-(ethoxyethyl)-1-piperazinyl)-phenyl)-benzimidazole-5-carboxylate (**10d**);
Benzyl 1-(3-(4-(methoxyethyl)-1-piperazinyl)-phenyl)-benzimidazole-5-carboxylate (**10e**);
Methylcarbamoylmethyl 1-(3-(4-(methoxyethyl)-1-piperazinyl)-phenyl)-benzimidazole-5-carboxylate (**10f**);
Ethylcarbamoylmethyl 1-(3-(4-(methoxyethyl)-1-piperazinyl)-phenyl)-benzimidazole-5-carboxylate (**10g**);
2-Dimethylaminoethyl 1-(3-(4-(methoxyethyl)-1-piperazinyl)-phenyl)-benzimidazole-5-carboxylate (**10h**);

or an N-oxide thereof, or a pharmaceutically acceptable salt thereof.

Definition of Substituents

In the context of this invention an alkyl group designates a univalent saturated, straight or branched hydrocarbon chain. The hydrocarbon chain preferably contain of from one to six carbon atoms ($C_{1-6}$-alkyl), including pentyl, isopentyl, neopentyl, tertiary pentyl, hexyl and isohexyl. In a preferred embodiment alkyl represents a $C_{1-4}$-alkyl group, including butyl, isobutyl, secondary butyl, and tertiary butyl. In another preferred embodiment of this invention alkyl represents a $C_{1-3}$-alkyl group, which may in particular be methyl, ethyl, propyl or isopropyl.

In the context of this invention an alkenyl group designates a carbon chain containing one or more double bonds, including di-enes, tri-enes and poly-enes. In a preferred embodiment the alkenyl group of the invention comprises of from two to six carbon atoms ($C_{2-6}$-alkenyl), including at least one double bond. In a most preferred embodiment the alkenyl group of the invention is ethenyl; 1- or 2-propenyl; 1-, 2- or 3-butenyl, or 1,3-butdienyl; 1-, 2-, 3-, 4- or 5-hexenyl, or 1,3-hexdienyl, or 1,3,5-hextrienyl.

In the context of this invention an alkynyl group designates a carbon chain containing one or more triple bonds, including di-ynes, tri-ynes and poly-ynes. In a preferred embodiment the alkynyl group of the invention comprises of from two to six carbon atoms ($C_{2-6}$-alkynyl), including at least one triple bond. In its most preferred embodiment the alkynyl group of the invention is ethynyl; 1-, or 2-propynyl; 1-, 2-, or 3-butynyl, or 1,3-butdiynyl; 1-,2-,3-, 4-pentynyl, or 1,3-pentdiynyl; 1-,2-,3-,4-, or 5-henynyl, or 1,3-hexdiynyl or 1,3,5-hextriynyl.

Alkoxy means O-alkyl, wherein alkyl is as defined above.

Alkoxyalkyl means alkoxy as above and alkyl as above, meaning for example, methoxymethyl.

Pharmaceutically Acceptable Salts

The chemical compound of the invention may be provided in any form suitable for the intended administration. Suitable forms include pharmaceutically (i.e. physiologically) acceptable salts of the chemical compound of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Examples of pharmaceutically acceptable cationic salts of a chemical compound of the invention include, without limitation, the sodium, the potassium, the calcium, the magnesium, the zinc, the aluminium, the lithium, the choline, the lysine, and the ammonium salt, and the like, of a chemical compound of the invention containing an anionic group. Such cationic salts may be formed by procedures well known and described in the art.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkylonium salts.

The chemical compound of the invention may be provided in dissoluble or indissoluble forms together with a pharmaceutically acceptable solvent such as water, ethanol, and the like. Dissoluble forms may also include hydrated forms such as the monohydrate, the dihydrate, the hemihydrate, the trihydrate, the tetrahydrate, and the like. In general, the dissoluble forms are considered equivalent to indissoluble forms for the purposes of this invention.

Steric Isomers

The chemical compounds of the present invention may exist in (+) and (-) forms as well as in racemic forms

(±). The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or l- (tartrates, mandelates, or camphorsulphonate) salts for example.

The chemical compounds of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by Jaques J, Collet A, & Widen S in "Enantiomers, Racemates, and Resolutions", John Wiley and Sons, New York (1981).

Optical active compounds can also be prepared from optical active starting materials.

Moreover, some of the chemical compounds of the invention having an alkenyl group, may exist in two forms, syn- and anti-form (Z- and E-form), depending on the arrangement of the substituents around the -C=C- double bond. A chemical compound of the present invention may thus be the syn- or the anti-form (Z- and E-form), or it may be a mixture hereof.

N-oxides

In the context of this invention an N-oxide designates an oxide derivative of a nitrogen containing compound, e.g. N-containing heterocyclic compounds capable of forming such N-oxides, and compounds holding one or more amino groups. For example, the N-oxide of a compound containing a pyridyl may be the 1-oxy-pyridin-2, -3 or -4-yl derivative.

N-oxides of the compounds of the invention may be prepared by oxidation of the corresponding nitrogen base using a conventional oxidizing agent such as hydrogen peroxide in the presence of an acid such as acetic acid at an elevated temperature, or by reaction with a peracid such as peracetic acid in a suitable solvent, e.g. dichloromethane, ethyl acetate or methyl acetate, or in chloroform or dichloromethane with 3-chloroperoxybenzoic acid.

Labelled Compounds

The compounds of the invention may be used in their labelled or unlabelled form. In the context of this invention "label" stands for the binding of a marker to the compound of interest that will allow easy quantitative detection of said compound.

The labelled compounds of the invention may be useful as diagnostic tools, radio tracers, or monitoring agents in various diagnostic methods, and for *in vivo* receptor imaging.

The labelled isomer of the invention preferably contains at least one radionuclide as a label. Positron emitting radionuclides are all candidates for usage. In the context of this invention the radionuclide is preferably selected from $^{2}H$ (deuterium), $^{3}H$ (tritium), $^{13}C$, $^{14}C$, $^{131}I$, $^{125}I$, $^{123}I$, and $^{18}F$.

The physical method for detecting the labelled isomer of the present invention may be selected from Position Emission Tomography (PET), Single Photon Imaging Computed Tomography (SPECT), Magnetic Resonance Spectroscopy (MRS), Magnetic Resonance Imaging (MRI), and Computed Axial X-ray Tomography (CAT), or combinations thereof.

**Methods of Preparation**

The chemical compounds of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

The compounds of this invention may exist in unsolvated as well as in solvated forms with pharmaceutically acceptable solvents such as water, ethanol and the like. In general, the solvated forms are considered equivalent to the unsolvated forms for the purposes of this invention.

**Pharmaceutical Compositions**

In another aspect the invention provides novel pharmaceutical compositions comprising a therapeutically effective amount of a compound of the invention.

While a compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising a compound of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

Pharmaceutical compositions of the invention may be those suitable for oral, rectal, bronchial, nasal, pulmonal, topical (including buccal and sub-lingual), transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The chemical compound of the invention, together with a conventional adjuvant, carrier, or diluent, may thus be placed into the form of pharmaceutical compositions and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The chemical compound of the present invention can be administered in a wide variety of oral and parenteral dosage forms. It will be obvious to those skilled in the art that the following dosage forms may comprise, as the active component, either a chemical compound of the invention or a pharmaceutically acceptable salt of a chemical compound of the invention.

For preparing pharmaceutical compositions from a chemical compound of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid, which is in a mixture with the finely divided active component.

In tablets, the active component is mixed with the carrier having the necessary binding capacity in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify.

Compositions suitable for vaginal administration may be presented as pessaries, tampons, creams, gels, pastes, foams or sprays containing in addition to the active ingredient such carriers as are known in the art to be appropriate.

Liquid preparations include solutions, suspensions, and emulsions, for example, water or water-propylene glycol solutions. For example, parenteral injection liquid preparations can be formulated as solutions in aqueous polyethylene glycol solution.

The chemical compound according to the present invention may thus be formulated for parenteral administration (e.g. by injection, for example bolus injection or continuous infusion) and may be presented in unit dose form in ampoules,

pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired.

Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations, intended for conversion shortly before use to liquid form preparations for oral administration. Such liquid forms include solutions, suspensions, and emulsions. In addition to the active component such preparations may comprise colorants, flavours, stabilisers, buffers, artificial and natural sweeteners, dispersants, thickeners, solubilizing agents, and the like.

For topical administration to the epidermis the chemical compound of the invention may be formulated as ointments, creams or lotions, or as a transdermal patch. Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co., Easton, PA).

A therapeutically effective dose refers to that amount of active ingredient, which ameliorates the symptoms or condition. Therapeutic efficacy and toxicity, e.g. $ED_{50}$ and $LD_{50}$, may be determined by standard pharmacological procedures in cell cultures or experimental animals. The dose ratio between therapeutic and toxic effects is the therapeutic index and may be expressed by the ratio $LD_{50}/ED_{50}$. Pharmaceutical compositions exhibiting large therapeutic indexes are preferred.

The dose administered must of course be carefully adjusted to the age, weight and condition of the individual being treated, as well as the route of administration, dosage form and regimen, and the result desired, and the exact dosage should of course be determined by the practitioner.

The actual dosage depend on the nature and severity of the disease being treated, and is within the discretion of the physician, and may be varied by titration of the dosage to the particular circumstances of this invention to produce

the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 μg/kg i.v. and 1 μg/kg p.o. The upper limit of the dosage range is presently considered to be about 10 mg/kg i.v. and 100 mg/kg p.o. Preferred ranges are from about 0.1 μg/kg to about 10 mg/kg/day i.v., and from about 1 μg/kg to about 100 mg/kg/day p.o.

**Biological Activity**

The compounds of the invention are particularly useful as anaesthetics and/or pre-anaesthetics, for inducing and maintaining anaesthesia, as sedatives, as muscle relaxants, and for combating febrile convulsions in children, status epilepticus, for use to patients constrained to a respirator.

The compounds of the invention show a short duration of action, they are water soluble at therapeutic relevant doses, and are particular well suited for intravenous administration.

The compounds of the invention may also be used by veterinarians.

The compounds of the invention show high to moderate affinity for the benzodiazepine receptor as measured by displacement at $^{3}$H-flunitrazepam *in vitro* as well as *in vivo.* The most preferred compounds are full agonists i.e. they exert a high maximal effect in the seizure test as described in the application.

Preferred compounds are full agonists on the $GABA_A$ receptor complex, e.g. as measured by the anticonvulsant activity in the ptz-test as described in Test method 2.

The compounds of the invention show half-lives of below 30 minutes, which allows for a short duration of action. Preferred half-lives are in the range of from about 30 seconds to about 20 minutes. Most preferred half-lives are in the range of from about 2 to about 5 minutes.

The preferred compounds induce a rapid onset of anaesthesia, i.e. in less than 1-2 minutes. Most preferred is an onset of anaesthesia in less than 1 minute.

Awakening from anaesthesia following a bolus injection (i.v.), or following the attenuation of an infusion, should occur within a short period of time, i.e. of from about 5 to about 30 minutes, preferably of from about 5 to about 10 minutes, after which time the patient should normalise rapidly, i.e. in less than 40 minutes, preferably in less than 20 minutes, as measured from awakening.

The compounds of this invention can be used together with analgetic compounds such as Remifentanile, Fentanyl, or other opiods.

**EXAMPLES**

The invention is further illustrated with reference to the following examples, which are not intended to be in any way limiting to the scope of the invention as claimed.

**Example 1**

**Method A:**

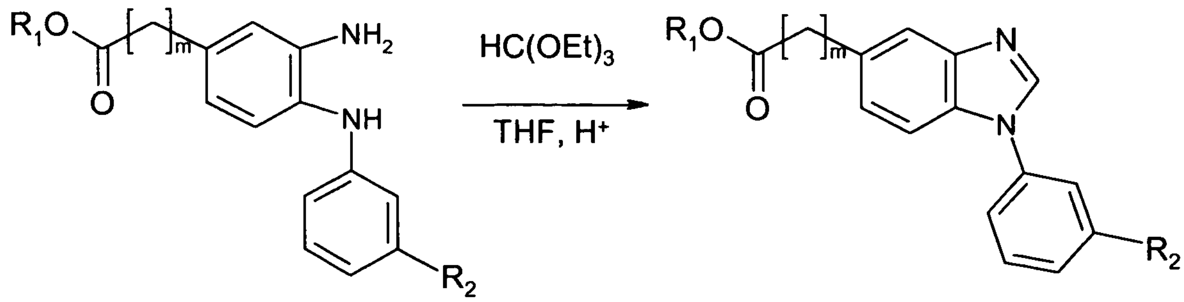


**2a-i** **1a-i**

The benzimidazoles of Table 1 were all prepared according to the above scheme.

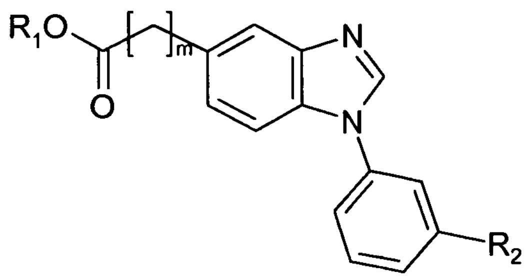

**Table 1**

| Comp. No. | $R_1$ | $R_2$ | m | Mp (˚C) | Yield (%) | Starting material | Salt |
|---|---|---|---|---|---|---|---|
| **1a** | $MeO(CH_2)_2$ | 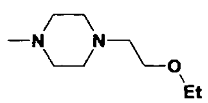 | 0 | A | 52 | **2a** | HCl |
| **1b** | $HO(CH_2)_2$ | 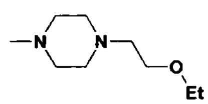 | 0 | A | 47 | **2b** | HCl |
| **1c** | $Me(CH_2)_3$ | 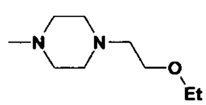 | 0 | 92-95 | 54 | **2c** | HCl |
| **1d** | $(CH_3)_2CHCH_2$ | 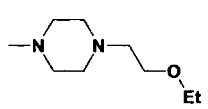 | 0 | 154-160 | 58 | **2d** | HCl |
| **1e** | $MeO(CH_2)_2$ | 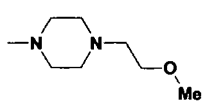 | 0 | 173-178 | 45 | **2e** | HCl |

(continued)

| Comp. No. | $R_1$ | $R_2$ | m | Mp (°C) | Yield (%) | Starting material | Salt |
|---|---|---|---|---|---|---|---|
| **1f** | $HO(CH_2)_2$ | 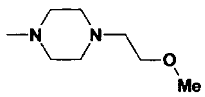 | 0 | 159-162 | 38 | **2f** | HCl |
| **1g** | $Me(CH_2)_3$ | 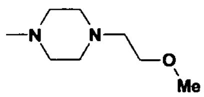 | 0 | 148-152 | 59 | **2g** | HCl |
| **1h** | $(CH_3)_2CHCH_2$ | 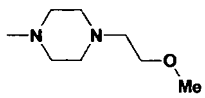 | 0 | 159-163 | 25 | **2h** | HCl |
| **1i** | Me | 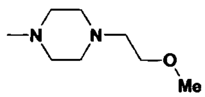 | 1 | A | 60 | **2i** | - |
| **1j** | $HO(CH_2)_2$ | 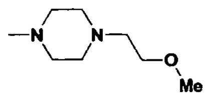 | 1 | A | 88 | **1i** | HCl |

The yield is given for a total of 3 steps starting from **4a-b** and **5a-e.** A few these compounds were hygroscopic solids, therefore no melting points were recorded. Instead these compounds were verified by exact mass determination. Results are given for compounds **1a, b, i** and **j** below.

<u>General procedure for the preparation of **1a-i:**</u>

A mixture of **2a-i** 1 eqv., triethylorthoformate 2 eqv. and a catalytic amount of p-toluenesulfonic acid in tetrahydrofurane (10 ml) was heated to reflux for 30 min. The cooled mixture was evaporated in vacuo, then dissolved in ethyl acetate and washed with sat. $NaHCO_3$ aq.. The organic phase was dried over magnesium sulphate and concentrated under reduced pressure. The residue was purified by column-chromatography on silica gel using $CH_2Cl_2$/ MeOH / $NH_3$aq. (9:1:1%) as an eluent.
The collected fractions were evaporated to give the desired product as a free base. Subsequently, the product was precipitated from THF as the hydrochloride by addition of a 1M etheral hydrogen chloride to the solution.
The following compounds were prepared in analogy with the above procedure:

<u>2-Methoxyethyl 1-(3-(4-(ethoxyethyl)-1-piperazinyl)-phenyl)-benzimidazole-5-carboxylate</u> **(1a).** Calc: $C_{25}H_{33}N_4O_4$ (M+H$^+$) = 453.2502. Found: $C_{25}H_{33}N_4O_4$ (M+H$^+$) = 453.2499.

<u>2-Hydroxyethyl 1-(3-(4-ethoxyethyl)-1-piperazinyl)-phenyl)-benzimidazole-5-carboxylate (**1b**)</u>. Calc: $C_{24}H_{31}N_4O_4$ (M+H$^+$) = 439.2345. Found: $C_{24}H_{31}N_4O_4$ (M+H$^+$) = 439.2348.

<u>*n*-Butyl 1-(3-(4-(ethoxyethyl)-1-piperazinyl)-phenyl)-benzimidazole-5-carboxylate</u> **(1c).**

<u>*iso*-Butyl 1-(3-(4-(ethoxyethyl)-1-piperazinyl)-phenyl)-benzimidazole-5-carboxylate</u> **(1d).**

<u>2-Methoxyethyl 1-(3-(4-(methoxyethyl)-1-piperazinyl)-phenyl)-benzimidazole-5-carboxylate</u> (**1e**).

<u>2-Hydroxvethyl 1-(3-(4-(methoxvethyl)-1-piperazinyl)-phenyl)-benzimidazole-5-carboxylate</u> (**1f**).

<u>*n*-Butyl 1-(3-(4-(methoxyethyl)-1-piperazinyl)-phenyl)-benzimidazole-5-carboxylate</u> (**1g**).

<u>*iso*-Butyl 1-(3-(4-(methoxyethyl)-1-piperazinyl)-phenyl)-benzimidazole-5-carboxylate</u> (**1h**).

<u>5-(methoxycarbonylmethyl) 1-(3-(4-(methoxyethyl)-1-piperazinyl)-phenyl)-benzimidazole</u> (**1i**). Calc: $C_{23}H_{29}N_4O_3$

(M+H+) = 409.2240. Found: $C_{23}H_{29}N_4O_3$ $(M+H^+)$ = 409.2222.

5-(2-hydroxyethoxycarbonylmethyl) 1-(3-(4-(methoxyethyl)-1-piperazinyl)-phenyl)-benzimidazole (**1j**): This compound was prepared from **1i** by transesterification. 0.2 g of **1i** was dissolved in 5 ml ethyleneglycol and heated to 100˚C overnight. The resulting cooled mixture was taken up in EtOAc and washed with $H_2O$. The organic phase was dried $MgSO_4$ and evaporated in vacuo. The compound was purified by column chromathography using $CH_2Cl_2$/MeOH/$NH_3$aq. (9:1:1 %) as an eluent. The product was dissolved in THF and an ethereal solution of hydrochloric acid was added. The resulting hydrochloride of **1j** was filtered of. Yield: 85%. Calc: $C_{24}H_{31}N_4O_4$ $(M+H^+)$ = 439.2345. Found: $C_{24}H_{31}N_4O_4$ $(M+H^+)$ = 439.2363.

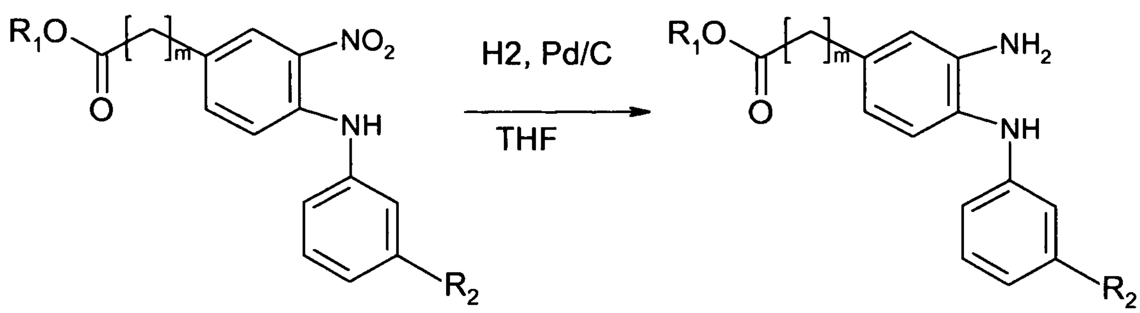


**3a-i** **2a-i**

The diamines of Table 2 were all prepared quantitatively by hydrogenation of the corresponding nitroanilines (**3**), according to the above scheme.

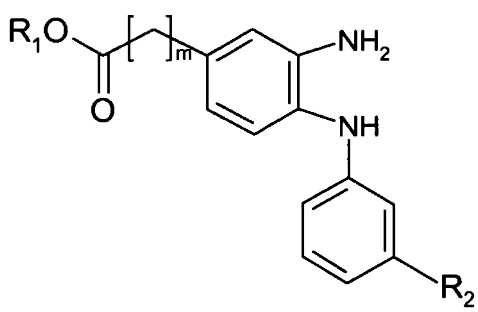

**Table 2**

| Comp. No | $R_1$ | $R_2$ | m | Starting material |
|---|---|---|---|---|
| **2a** | $MeO(CH_2)_2$ | 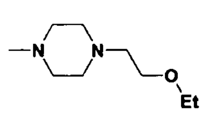 | 0 | **3a** |
| **2b** | $HO(CH_2)_2$ | 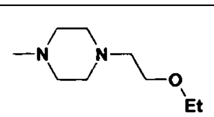 | 0 | **3b** |
| **2c** | $Me(CH_2)_3$ | 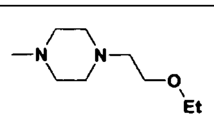 | 0 | **3c** |

(continued)

| Comp. No | $R_1$ | $R_2$ | m | Starting material |
|---|---|---|---|---|
| **2d** | $(CH_3)_2CHCH_2$ | 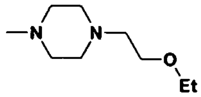 | 0 | **3d** |
| **2e** | $MeO(CH_2)_2$ | 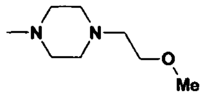 | 0 | **3e** |
| **2f** | $HO(CH_2)_2$ | 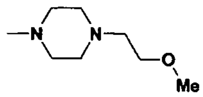 | 0 | **3f** |
| **2g** | $Me(CH_2)_3$ | 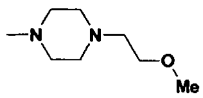 | 0 | **3g** |
| **2h** | $(CH_3)_2CHCH_2$ | 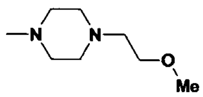 | 0 | **3h** |
| **2i** | Me | 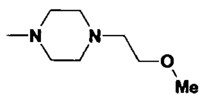 | 1 | **3i** |

General procedure for the hydrogenation of **3a-i:**

**3a-i** was suspended in tetrahydrofurane. Palladium catalyst (50 mg, 5% on activated carbon) was added and the mixture was hydrogenated at ambient pressure until the hydrogen uptake had ceased. The mixture was filtered through celite and the filtrate was evaporated to dryness to leave **2a-i,** quantitatively.

The following compounds were prepared in according to the above mentioned procedure.

2-Methoxyethyl 3-amino-4-(3-((1-ethoxyethyl-4-piperazinyl)-phenylamino)-benzoate (**2a**).

2-Hydroxyethyl 3-amino-4-(3-(1-(ethoxyethyl-4-piperazinyl)-phenylamino)-benzoate (**2b**).

*n*-Butyl 3-amino-4-(3-((1-ethoxyethyl-4-piperazinyl)-phenylamino)-benzoate (**2c**).

*iso*-Butyl 3-amino-4-(3-((1-ethoxyethyl-4-piperazinyl)-phenylamino)-benzoate (**2d**).

2-Methoxyethyl 3-amino-4-(3-((1-methoxyethyl-4-piperazinyl)-phenylamino)-benzoate **(2e).**

2-Hydroxyethyl 3-amino-4-(3-(1-(methoxyethyl)-4-piperazinyl)-phenylamino)-benzoate (**2f**).

*n*-Butyl 3-amino-4-(3-((1-methoxyethyl-4-piperazinyl)-phenylamino)-benzoate (**2g**).

*iso*-Butyl 3-amino-4-(3-((1-methoxyethyl-4-piperazinyl)-phenylamino)-benzoate (**2h**).

5-Methoxycarbonylmethyl 2-(3-((1-methoxyethyl-4-piperazinyl)-phenylamino)-aniline (**2i**).

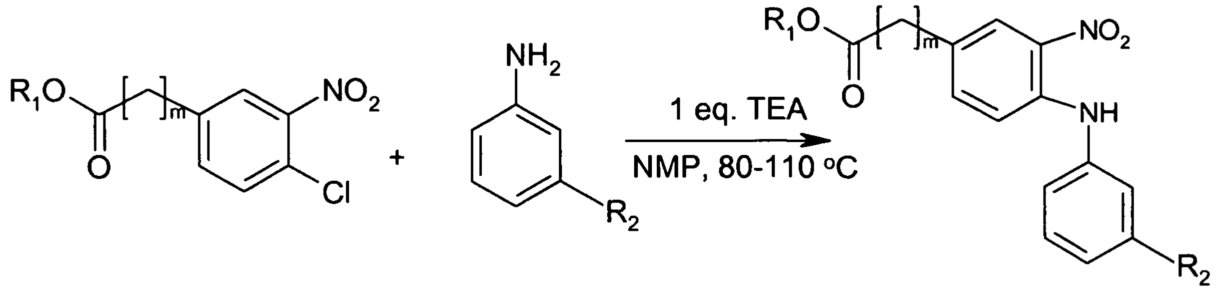


**5a-e** **4a-b** **3a-i**

The nitroanilines of Table 3 were prepared by reaction of 4-chloro-3-nitrobenzoates **5** with substituted anilines (**4**), according to the above scheme.

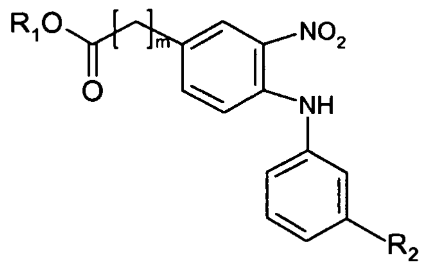

**Table 3**

| Comp. No. | $R_1$ | $R_2$ | m | Starting material |
|---|---|---|---|---|
| **3a** | $MeO(CH_2)_2$ | 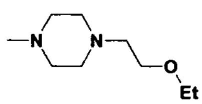 | 0 | **4a, 5a** |
| **3b** | $HO(CH_2)_2$ | 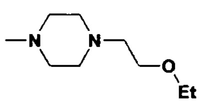 | 0 | **4a, 5b** |
| **3c** | $Me(CH_2)_3$ | 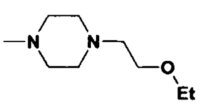 | 0 | **4a, 5c** |
| **3d** | $(CH_3)_2CHCH_2$ | 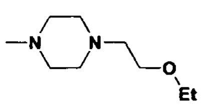 | 0 | **4a, 5d** |
| **3e** | $MeO(CH_2)_2$ | 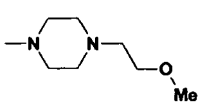 | 0 | **4b, 5a** |
| **3f** | $HO(CH_2)_2$ | 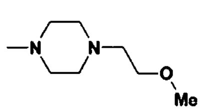 | 0 | **4b, 5b** |

(continued)

| Comp. No. | $R_1$ | $R_2$ | m | Starting material |
|---|---|---|---|---|
| **3g** | $Me(CH_2)_3$ | 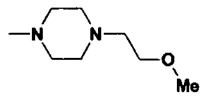 | 0 | **4b, 5c** |
| **3h** | $(CH_3)_2CHCH_2$ | 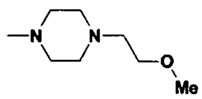 | 0 | **4b, 5d** |
| **3i** | Me | 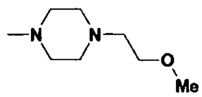 | 1 | **4b, 5e** |

General procedure for the preparation of compounds **3a-i:**

A mixture of **5a-e** 1 eqv., **4a-b** 1 eqv. and triethylamine 1 eqv. in NMP (10 ml) was heated to 110˚C overnight. The cooled mixture was partitioned between water and ethyl acetate. The phases were separated and the aqueous phase was extracted with ethyl acetate. The combined organic phases were washed with brine, dried over magnesium sulphate and concentrated under reduced pressure. The residue was purified by column-chromatography on silica gel using a mixture of ethyl acetate and petroleum ether (1:1 v/v) as the eluent.

The following compounds were prepared in analogy with the above-mentioned procedure.

2-Methoxyethyl 3-nitro-4-(3-(1-(ethoxyethyl-4-piperazinyl)-phenylamino)-benzoate **(3a).**

2-Hydroxyethyl 3-nitro-4-(3-(1-(ethoxyethyl-4-piperazinyl)-phenylamino)-benzoate **(2b).**

*n*-Butyl 3-nitro-4-(3-((1-ethoxyethyl-4-piperazinyl)-phenylamino)-benzoate **(2c)**

*iso*-Butyl 3-nitro-4-(3-((1-ethoxyethyl-4-piperazinyl)-phenylamino)-benzoate **(2d)**

2-Methoxyethyl 3-nitro-4-(3-((1-methoxyethyl-4-piperazinyl)-phenylamino)-benzoate **(2e).**

2-Hydroxyethyl 3-nitro-4-(3-(1-(methoxyethyl)-4-piperazinyl)-phenylamino)-benzoate **(2f).**

*n*-Butyl 3-nitro-4-(3-((1-methoxyethyl-4-piperazinyl)-phenylamino)-benzoate **(2g).**

*iso*-Butyl 3-nitro-4-(3-((1-methoxyethyl-4-piperazinyl)-phenylamino)-benzoate (**2h**).

5-Methoxycarbonylmethyl 2-(3-((1-methoxyethyl-4-piperazinyl)-phenylamino)-nitrobenzene (**2i**).

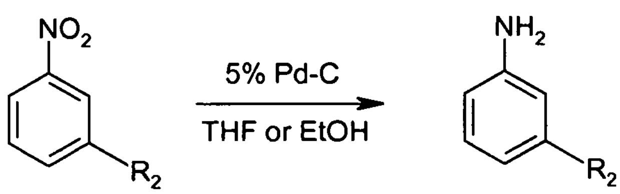


**6a-b** **4a-b**

The substituted anilines of Table 4 were prepared by hydrogenation of the corresponding nitro compounds **6a-b** as exemplified by compound **4a** below.

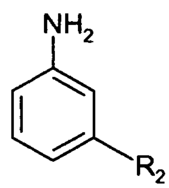

**Table 4**

| Comp. No. | $R_2$ | Starting material |
|---|---|---|
| **4a** | 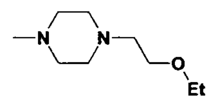 | **6a** |
| **4b** | 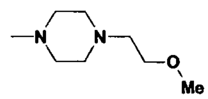 | **6b** |

General procedure for the hydrogenation of compounds **6a-b:**

To a solution of **6a** or **6b** in abs. ethanol (50 ml) was added palladium catalyst (100 mg, 5% Pd on activated carbon) and the mixture was hydrogenated at ambient pressure until the hydrogen uptake had ceased. Filtration through celite and evaporation of solvent left **4a** or **4b,** quantitatively.

The following compounds were prepared according to the above mentioned procedure:

1-Ethoxyethyl-4-(3-aminophenyl)-piperazine **(4a).**

1-Methoxyethyl-4-(3-aminophenyl)-piperazine **(4b).**

The 3-nitro-4-chlorobenzoicacid esters **5a-d** were prepared by esterification of the corresponding benzoic acids by the method mentioned below.

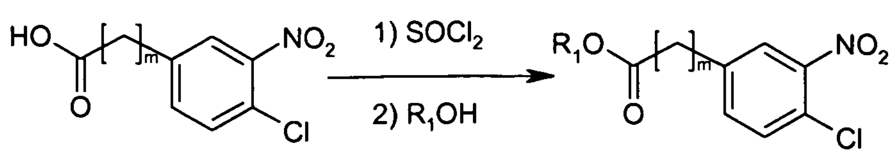


**Table 5**

| Comp No. | $R_1$ | m | Yield % | X | Starting material |
|---|---|---|---|---|---|
| **5a** | $MeO(CH_2)_2$ | | 0 62 | Cl | 4-chloro-3-nitrobenzoic acid |
| **5b** | $HO(CH_2)_2$ | 0 | 88 | Cl | 4-chloro-3-nitrobenzoic acid |
| **5c** | $Me(CH_2)_3$ | 0 | 96 | Cl | 4-chloro-3-nitrobenzoic acid |
| **5d** | $(CH_3)_2CHCH_2$ | 0 | 95 | Cl | 4-chloro-3-nitrobenzoic acid |
| **5e** | Me | 1 | 55 | F | 4-fluorophenylacetic acid |

General procedure for the preparation of **5a-d:**

A mixture of acid (10g) and thionylchloride (50 ml) was heated to reflux overnight. The excess of thionylchloride

was removed by evaporation and alcohol (50 ml) was added. The resulting mixture was stirred at 80˚C for 4 hours. The cooled solution was diluted with water (500 ml) and extracted with ethyl acetate (2 x 100 ml). The organic extract was washed with $NaHCO_3$ sat. and dried over magnesium sulphate and concentrated under reduced pressure. This gave the corresponding esters of a relatively high purity.

The following compounds were prepared according to the above mentioned procedure:

2-Methoxyethyl 4-chloro-3-nitrobenzoate **(5a).**

2-Hydroxyethyl 4-chloro-3-nitrobenzoate **(5b).**

*n*-Butyl 4-chloro-3-nitrobenzoate **(5c)**.

*iso*-Butyl 4-chloro-3-nitrobenzoate **(5d).**

Methoxycarbonylmethyl-4-chloro-3-nitrobenzene **(5e)**

4-fluorophenylacetic acid (6g, 38.9 mmol) was suspended in 50 ml $H_2SO_4$ conc. And cooled to 0˚C. To this suspension was added dropwise 1.75 ml $HNO_3$ during 30 min. and then the reaction mixture was striired at 0˚C for another 3h. The yellow mixture was poured into is-water and the corresponding white crystals 4-fluoro-3-nitrophenylacetic acid 4.55g, 59% was collected and dried.

4-fluoro-3-nitrophenylacetic acid (4.55g, 22.9 mmol) was suspended in 50 ml MeOH and added 0.15 ml $H_2SO_4$ followed by reflux for 2h. After cooling the reaction mixture was poured into water and then added $NaHCO_3$ until pH>7. The extraction with EtOAc, drying with MgSO4 and evaporation in vacuo gave **5e.** Yield 4.25g, 87%.

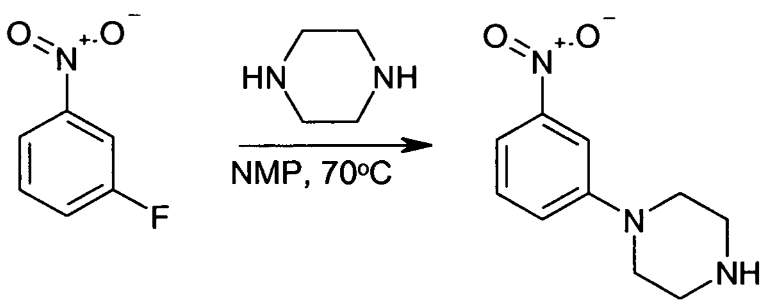


1-(3-Nitrophenyl)-piperazine

A suspension of 3-fluoronitrobenzene (23 ml; 0.21 mol) and piperazine (55.5 g; 0.64 mol) in anhydrous NMP (30 ml) was heated to 70˚C for five days. The cooled mixture was diluted with water (250 ml) and extracted with dichloromethane. The combined extracts were dried over magnesium sulphate and concentrated under reduced pressure. The residue was purified by column-chromatography on silica gel eluting subsequently with mixtures of ethyl acetate and methanol (4:1 v/v) and (1:1 v/v) to leave the desired product as oily crystals (30.7 g; 71 %).

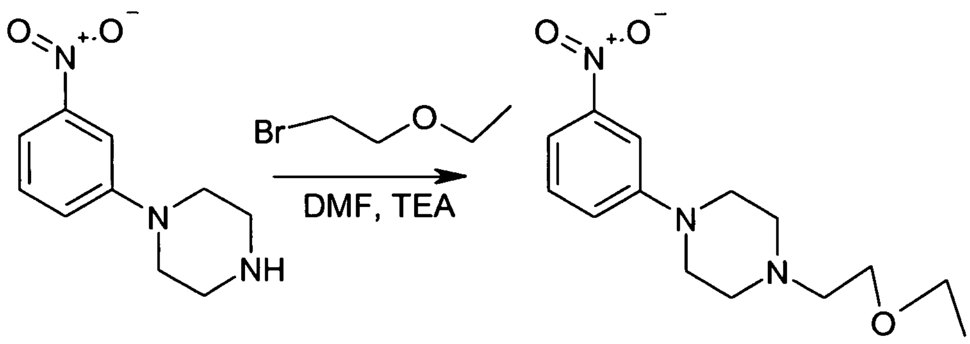


6a

Ethoxyethyl 2-(4-(3-nitrophenyl)-1-piperazine) **(6a)**

To a suspension of 1-(3-nitrophenyl)piperazine (8.18 g; 33.7 mmol) in DMF (80 ml) was added triethylamine (9.9 ml; 70.7 mmol) and the reaction was allowed to stir at RT for 30 min. Then bromoethylethyl ether (6 ml; 50 mmol) was added, the mixture was stirred at ambient temperature for 2h. the reaction was worked up by evaporating DMF followed by resuspension in EtOAc and washing with $NaHCO_3$ sat. The organic phase was dried and evaporated to give an yellowish oil. This was purified by column chromatography on silica gel using $CH_2Cl_2$/MeOH (8:2) as an eluent. Yield of **6a** (7.5 g; 81 %).

The following compounds were prepared in analogy with Compound **6a:**

Methoxyethyl 2-(4-(3-nitrophenyl)-1-piperazine) **(6b)** Yield 78%.

**Example 2**

**Method B**

The following compounds were synthesised according to the method mentioned below.

Compound **7a** was synthesised from 2-Methoxyethyl 1-(3-(4-methoxycarbonylmethyl-1-piperazinyl)-phenyl)-benzimidazole-5-carboxylate by dealkylation of the piperazine ring using the method by Kondo et al, J. Med. Chem., 1989, 32 (3), 679-682. 2-Methoxyethyl 1-(3-(4-methoxycarbonylmethyl-1-piperazinyl)-phenyl)-benzimidazole-5-carboxylate was synthesised according to International Patent Publication No. WO 00/78728, example 1b.

In the same way compound **7b** was synthesised by dealkylation of 2-methoxyethyl 1-(3-(1-methoxycarbonylmethyl-4-piperidinyl)-phenyl)-benzimidazole-5-carboxylate using the same literature method as mentioned for **7a.** 2-methoxyethyl 1-(3-(1-methoxycarbonylmethyl-4-piperidinyl)-phenyl)-benzimidazole-5-carboxylate was synthesised according to International Patent Publication No. WO 02/050057, Example 1d.

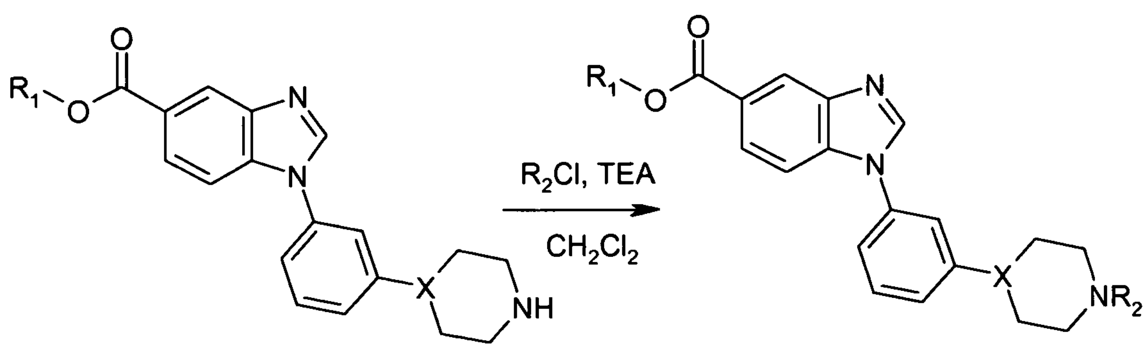


**7a,b** **8a-d**

**Table 6**

| **Compound nr.** | $R_1$ | $R_2$ | X | Yield | **Starting material** | Salt |
|---|---|---|---|---|---|---|
| | | | | | | |
| | | | | | | |
| | | | | | | |
| **8d** | $MeO(CH_2)_2$ | 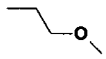 | C | 43 | **7b** | formiate |
| | | | | | | |

<u>General procedure for the preparation of compounds **8a-d:**</u>

To a solution of **7a** or **7b** in $CH_2Cl_2$ was added triethylamine 2 eqv. Followed by 2 eq. of $R_2Cl$. And stirring was maintained overnight at ambient temperature. The organic phase was washed with water, dried (MgSO4) and concentrated *in vacuo*. The crude products were purified by preparative LCMS using a gradient 20% B to 95% B in 13 min. A) 5mM $(NH_4)HCO_3/H_2O$ B) Acetonitrile. The collected fractions were pooled and acidified with formic acid before evaporation of the solvent. This gave **8a-c** as white solids.

The following compound was prepared by the above-mentioned method:

<u>2-Methoxyethyl 1-(3-(1-methoxvethyl-4-piperidinyl)-phenyl)-benzimidazole-5-carboxylate</u> **(8d).** Calc: $C_{25}H_{32}N_3O_4$ $(M+H^+)$ = 438.2393. Found: $C_{25}H_{32}N_3O_4$ $(M+H^+)$ = 438.2382.

**Example 3**

**Method C**

**1a or 1e**

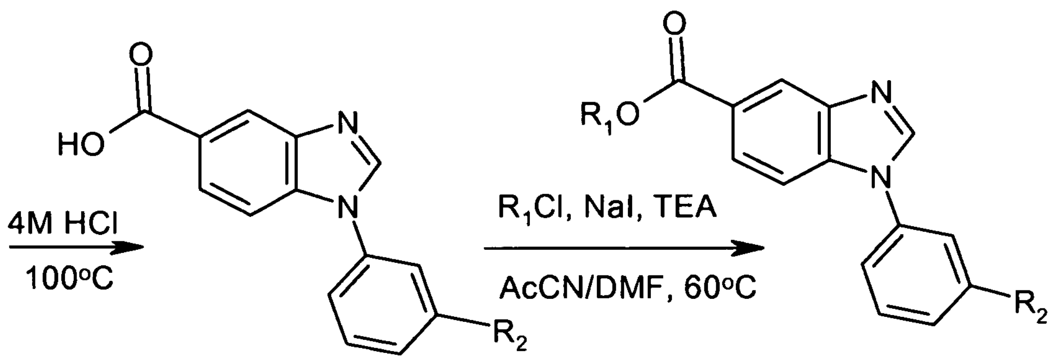


**9a,b** **10a-h**

**Table 7**

| **Compound No.** | $R_1$ | $R_2$ | **Yield %** | **Starting material** | **Salt** |
|---|---|---|---|---|---|
| **10a** | $PhCH_2$ | 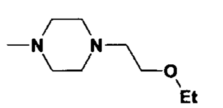 | 42 | **9a** | HCl |

(continued)

| Compound No. | $R_1$ | $R_2$ | Yield % | Starting material | Salt |
|---|---|---|---|---|---|
| **10b** | $MeNHCOCH_2$ | 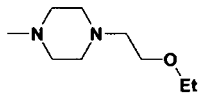 | 25 | **9a** | HCl |
| **10c** | $EtNHCOCH_2$ | 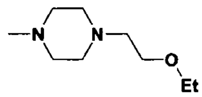 | 50 | **9a** | HCl |
| **10d** | $Me_2N(CH_2)_2$ | 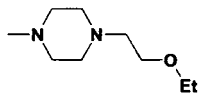 | 80 | **9a** | HCl |
| **10e** | $PhCH_2$ | 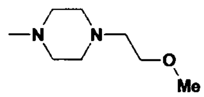 | 53 | **9b** | - |
| **10f** | $MeNHCOCH_2$ | 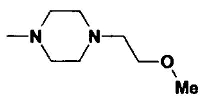 | 48 | **9b** | HCl |
| **10g** | $EtNHCOCH_2$ | 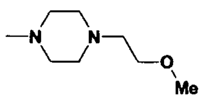 | 62 | **9b** | HCl |
| **10h** | $Me_2N(CH_2)_2$ | 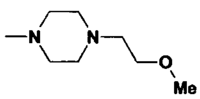 | 76 | **9b** | HCl |

General procedure for the preparation of compounds **9a** and **9b:**

Compound **1a** or **1e** was hydrolysed by dissolving the compound in 4M HCl and refluxed overnight. The reaction mixture was then cooled and evaporated in vacuo to give the crude acids **9a** and **9b,** respectively. No further purification was attempted at this point but the crude reaction mixture was used directly in the next step.

General procedure for the preparation of compounds **10a-h:**

The crude acid **9a** or **9b** was dissolved in acetonitrile / DMF 4:1 and then added triethylamine in excess (>3eq) and a small catalytic amound of NaI. The reaction mixture was stirred for 15 min after which the $R_1Cl$ (2-3 eq.) was added and the reaction was then heated to 60°C overnight. After cooling the acetonitrile was evaporated and the resulting mixture was taken up in DCM and washed with water. The organic phase was dried $MgSO_4$ and evaporated give oily products. Column chromathography on silica gel using $CH_2Cl_2$/ MeOH/ $NH_3$aq. (9:1:1 %) as an eluent gave the pure compounds **10a-h.**

The following compounds were prepared using the above method:

Benzyl 1-(3-(4-(ethoxyethyl)-1-piperazinyl)-phenyl)-benzimidazole-5-carboxylate **(10a).** Calc: $C_{29}H_{33}N_4O_3$ $(M+H^+)$ = 485.2553. Found: $C_{25}H_{33}N_4O_3$ $(M+H^+)$ = 485.2549.

Methylcarbamoylmethyl 1-(3-(4-(ethoxyethyl)-1-piperazinyl)-phenyl)-benzimidazole-5-carboxylate **(10b).** Calc: $C_{25}H_{35}N_5O_4$ $(M+H^+)$ = 466.2454. Found: $C_{25}H_{35}N_5O_4$ $(M+H^+)$ = 466.2445.

Ethylcarbamoylmethyl 1-(3-(4-(ethoxyethyl)-1-piperazinyl)-phenyl)-benzimidazole-5-carboxylate **(10c).** Mp. 153-154°C.

2-Dimethylaminoethyl 1-(3-(4-(ethoxyethyl)-1-piperazinyl)-phenyl)-benzimidazole-5-carboxylate **(10d).** Calc:

$C_{26}H_{36}N_5O_3$ (M+H+) = 466.2818. Found: $C_{26}H_{36}N_5O_3$ (M+H+) = 466.2811.

Benzyl 1-(3-(4-(methoxyethyl)-1-piperazinyl)-phenyl)-benzimidazole-5-carboxylate **(10e).** Mp. 63˚C.

Methylcarbamoylmethyl 1-(3-(4-(methoxyethyl)-1-piperazinyl)-phenyl)-benzimidazole-5-carboxylate **(10f).** Calc: $C_{24}H_{30}N_5O_4$ (M+H+) = 452.2298. Found: $C_{24}H_{30}N_5O_4$ (M+H+) = 452.2286.

Ethylcarbamoylmethyl 1-(3-(4-(methoxyethyl)-1-piperazinyl)-phenyl)-benzimidazole-5-carboxylate **(10g).** Calc: $C_{25}H_{32}N_5O_4$ (M+H+) = 466.2454. Found: $C_{25}H_{32}N_5O_4$ (M+H+) = 466.2459.

2-Dimethylaminoethyl 1-(3-(4-(methoxyethyl)-1-piperazinyl)-phenyl)-benzimidazole-5-carboxylate **(10h).** Calc: $C_{25}H_{34}N_5O_3$ (M+H+) = 452.2552. Found: $C_{25}H_{34}N_5O_3$ (M+H+) = 452.2646.

**TEST METHODS**

**Test method 1**

***In vitro* and *in vivo* Binding Activity**

The GABA recognition site and the benzodiazepine modulatory unit can selectively be labelled with $^3$H-muscimol and $^3$H-flunitrazepam, respectively.

**1A: *In vitro* inhibition of $^3$H-flunitrazepam ($^3$H-FNM) binding**

Tissue Preparation

Preparations are performed at 0-4˚C unless otherwise indicated. Cerebral cortex from male Wistar rats (150-200 g) is homogenised for 5-10 sec in 20 ml Tris-HCl (30 mM, pH 7.4) using an Ultra-Turrax homogeniser. The suspension is centrifuged at 27,000 x g for 15 min and the pellet is washed three times with buffer (centrifuged at 27,000 x g for 10 min). The washed pellet is homogenized in 20 ml of buffer and incubated on a water bath (37˚C) for 30 min to remove endogenous GABA and then centrifuged for 10 min at 27,000 x g. The pellet is then homogenized in buffer and centrifuged for 10 min at 27,000 x g. The final pellet is resuspended in 30 ml buffer and the preparation is frozen and stored at -20˚C.

Assay

The membrane preparation is thawed and centrifuged at 2˚C for 10 min at 27,000 x g. The pellet is washed twice with 20 ml 50 mM Tris-citrate, pH 7.1 using an Ultra-Turrax homogeniser and centrifuged for 10 min at 27,000 x g. The final pellet is resuspended in 50 mM Tris-citrate, pH 7.1 (500 ml buffer per g of original tissue), and then used for binding assays. Aliquots of 0.5 ml tissue are added to 25 μl of test solution and 25 μl of $^3$H-FNM (1 nM, final concentration), mixed and incubated for 40 min at 2˚C. Non-specific binding is determined using Clonazepam (1 μM, final concentration). After incubation the samples are added 5 ml of ice-cold buffer and poured directly onto Whatman GF/C glass fibre filters under suction and immediately washed with 5 ml ice-cold buffer. The amount of radioactivity on the filters is determined by conventional liquid scintillation counting. Specific binding is total binding minus non-specific binding.

Results

25-75% inhibition of specific binding must be obtained, before calculation of an $IC_{50}$. The test value will be given as $IC_{50}$ (the concentration (μM) of the test substance which inhibits the specific binding of $^3$H-FNM by 50%).

$$IC_{50} = (\text{applied test substance concentration, } \mu M) \times \frac{1}{\left(\frac{C_o}{C_x} - 1\right)}$$

where

$C_o$ is specific binding in control assays, and
$C_x$ is the specific binding in the test assay.
(The calculations assume normal mass-action kinetics).

**1B: *In vivo* inhibition of $^3$H-FNM binding**

Introduction

*In vitro* binding studies have demonstrated that the benzodiazepine [$^3$H]FNM binds selectively and with high-affinity to the $GABA_A$ receptor-ion channel complex. [$^3$H]FNM can also be used for *in vivo* receptor labelling studies in mouse. Accumulation of [$^3$H]FNM binding will occur all over the brain as $GABA_A$ receptors are widely distributed. The specific binding of [$^3$H]FNM can be partly or completely prevented by simultaneous or prior administration of pharmacologically active benzodiazepines or by some benzodiazepine-like compounds.

Method

All test substances used are solutions prepared in 10% TWEEN 80. Groups of three female NMRI mice (25 g) are injected i.v. via the tail vein with 5.0 μCi of [$^3$H]FNM in 0.2 ml saline. Fifteen min after injection with [$^3$H]FNM the test substance is administered i.v. Twenty min after injection with [$^3$H]FNM, mice are killed by decapitation, the forebrains rapidly excised and homogenized in 12 ml of ice-cold 50 mM Tris-citrate, pH 7.1 using an Ultra-Turrax homogenizer. Three aliquots of 1 ml are immediately filtered through GF/C glass fibre filters and washed with 2 x 5 ml of ice-cold buffer. The amounts of radioactivity on the filters and in 200 μl of the homogenate are determined by conventional scintillation counting. Groups of untreated mice serves as controls. To determine non-specific binding groups of mice are injected with Clonazepam (25 mg/kg) i.p. 10 min before [$^3$H]FNM injection. Specific binding is the amount of binding in controls minus the amount of binding in Clonazepam treated mice.

Results

The $ED_{50}$ value is determined from dose response curves. If only one dose of test substance is administered, the $ED_{50}$ value is calculated as follows, provided that the inhibition of specific binding is within the range of 25-75%.

$$ED_{50} = (\text{administered dose, mg/kg}) \times \frac{1}{\left(\frac{C_o}{C_x} - 1\right)}$$

where $C_o$ is specific binding in controls and $C_x$ is the specific binding in mice treated with test substance.

**Test method 2**

**PTZ Clonic Convulsions**

The purpose of this test is to show antagonism of clonic convulsions induced by pentylenetetrazol (PTZ). PTZ induces clonic convulsions in mice after i.v. infusion. Antagonism of PTZ-induced convulsions is a measure for the agonistic character of ligands for the benzodiazepine recognition site.

Procedure

Female NMRI mice (Bomholdtgaard, Ry), 20 g, 6 mice in each group are administered i.v. with vehicle or test substance. After five minutes the PTZ-solution is infused intravenously at a speed of 0.7 ml/minute through a cannula placed in the tail vein. The time from initiation of the infusion to appearance of clonic convulsions is recorded.
The dose of PTZ required for inducing convulsion in each mouse is calculated as PTZ/kg body weight. Means ±sd for each experimental group of 6 mice is calculated. $ED_{100}$ is calculated by linear regression expressing the dose increasing the PTZ threshold to 100 mg PTZ/kg.
The threshold of vehicle treated controls is in the range of 37-39 mg PTZ/kg. As a control in each series of experiments PTZ is infused into 6 vehicle treated mice.

**Test method 3**

**Evaluation of Efficacy**

Selected compounds exhibiting a promising profile in the above tests may be evaluated with respect to efficacy and duration of action and compared to prior art as follows.

Aqueous solutions of the test substances (50 mg/ml isotonic glucose) are administered to pigs (25-30 kg) as bolus injections. The pigs are observed with respect to the time of induction of anaesthesia, the duration of anaesthesia and the normalising time following awakening from anaesthesia.

**Claims**

1. A compound of general formula I:

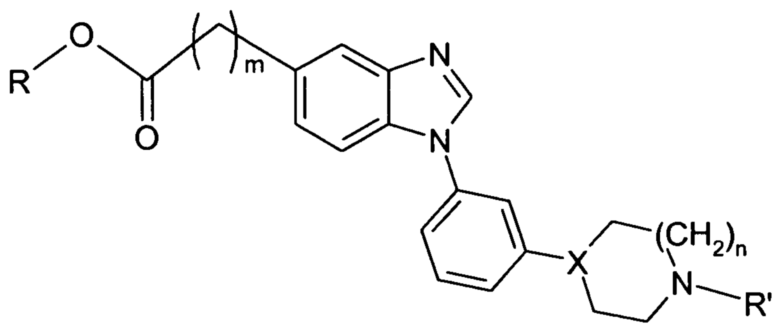

(I)

or an N-oxide thereof, or a pharmaceutically acceptable salt thereof, wherein R represents $C_{1-6}$-alkyl, hydroxy-$C_{1-6}$-alkyl, $C_{1-6}$-alkoxy-$C_{1-6}$-alkyl, $R^aR^bN$-$C_{1-6}$-alkyl, $R^aR^bN$-CO-$C_{1-6}$-alkyl, or phenyl-$C_{1-6}$-alkyl;
wherein $R^a$ and $R^b$ independently of each other represents hydrogen or $C_{1-6}$-alkyl;
R' represents $C_{1-6}$-alkoxy-$C_{1-6}$-alkyl, $C_{1-6}$-alkoxy-$C_{2-6}$-alkenyl or $C_{1-6}$-alkoxy-$C_{2-6}$-alkynyl;
m is 0 or 1;
n is 1;
X represents N or CH.

2. The compound of claim 1, wherein
X represents N.

3. The compound of claim 1, wherein
X represents CH.

4. The compound of any one of claims 1-3, wherein
R represents $C_{1-6}$-alkyl, hydroxy-$C_{1-6}$-alkyl, or $C_{1-6}$-alkoxy-$C_{1-6}$-alkyl.

5. The compound of any one of claims 1-3, wherein
R represents $R^aR^bN$-$C_{1-6}$-alkyl, $R^aR^bN$-CO-$C_{1-6}$-alkyl, or phenyl-$C_{1-6}$-alkyl; wherein $R^a$ and $R^b$ independently of each other represents hydrogen, methyl or ethyl.

6. The compound of any one of claims 1-5, wherein
R' represents $C_{1-6}$-alkoxy-$C_{1-6}$-alkyl.

7. The compound of claim 1, which is
2-Methoxyethyl 1-(3-(4-(ethoxyethyl)-1-piperazinyl)-phenyl)-benzimidazole-5-carboxylate **(1a);**
2-Hydroxyethyl 1-(3-(4-ethoxyethyl)-1-piperazinyl)-phenyl)-benzimidazole-5-carboxylate **(1b);**
*n*-Butyl 1-(3-(4-(ethoxyethyl)-1-piperazinyl)-phenyl)-benzimidazole-5-carboxylate **(1c);**
*iso*-Butyl 1-(3-(4-(ethoxyethyl)-1-piperazinyl)-phenyl)-benzimidazole-5-carboxylate **(1d);**
2-Methoxyethyl 1-(3-(4-(methoxyethyl)-1-piperazinyl)-phenyl)-benzimidazole-5-carboxylate **(1e);**

2-Hydroxyethyl 1-(3-(4-(methoxyethyl)-1-piperazinyl)-phenyl)-benzimidazole-5-carboxylate **(1f);**
*n*-Butyl 1-(3-(4-(methoxyethyl)-1-piperazinyl)-phenyl)-benzimidazole-5-carboxylate **(1g);**
*iso*-Butyl 1-(3-(4-(methoxyethyl)-1-piperazinyl)-phenyl)-benzimidazole-5-carboxylate **(1h);**
5-(methoxycarbonylmethyl) 1-(3-(4-(methoxyethyl)-1-piperazinyl)-phenyl)-benzimidazole **(1i);**
5-(2-hydroxyethoxycarbonylmethyl) 1-(3-(4-(methoxyethyl)-1-piperazinyl)-phenyl)-benzimidazole **(1j);**
2-Methoxyethyl 1-(3-(1-methoxyethyl-4-piperidinyl)-phenyl)-benzimidazole-5-carboxylate **(8d);**
Benzyl 1-(3-(4-(ethoxyethyl)-1-piperazinyl)-phenyl)-benzimidazole-5-carboxylate **(10a);**
Methylcarbamoylmethyl 1-(3-(4-(ethoxyethyl)-1-piperazinyl)-phenyl)-benzimidazole-5-carboxylate **(10b);**
Ethylcarbamoylmethyl 1-(3-(4-(ethoxyethyl)-1-piperazinyl)-phenyl)-benzimidazole-5-carboxylate **(10c);**
2-Dimethylaminoethyl 1-(3-(4-(ethoxyethyl)-1-piperazinyl)-phenyl)-benzimidazole-5-carboxylate **(10d);**
Benzyl 1-(3-(4-(methoxyethyl)-1-piperazinyl)-phenyl)-benzimidazole-5-carboxylate **(10e);**
Methylcarbamoylmethyl 1-(3-(4-(methoxyethyl)-1-piperazinyl)-phenyl)-benzimidazole-5-carboxylate **(10f);**
Ethylcarbamoylmethyl 1-(3-(4-(methoxyethyl)-1-piperazinyl)-phenyl)-benzimidazole-5-carboxylate **(10g);**
2-Dimethylaminoethyl 1-(3-(4-(methoxyethyl)-1-piperazinyl)-phenyl)-benzimidazole-5-carboxylate **(10h);**
or a pharmaceutically acceptable salt thereof.

**8.** A pharmaceutical composition containing a therapeutically effective amount of a compound according to any one of claims 1-7, or an N-oxide thereof, or a pharmaceutically acceptable salt thereof, together with at least one pharmaceutically acceptable carrier, excipient or diluent.

**9.** The use of a compound according to any one of claims 1-7, or an N-oxide thereof, or a pharmaceutically acceptable salt thereof, for the manufacture of a medicament for the treatment, prevention or alleviation of a disease or a disorder or a condition of a mammal, including a human, which disease, disorder or condition is fewer cramps or status epilepticus.

**10.** The use of a compound according to any one of claims 1-7, or an N-oxide thereof, or a pharmaceutically acceptable salt thereof, for the manufacture of a medicament for inducing anaesthesia, pre-anaesthesia, muscle relaxation, or sedation.

## Patentansprüche

**1.** Verbindung der allgemeinen Formel I:

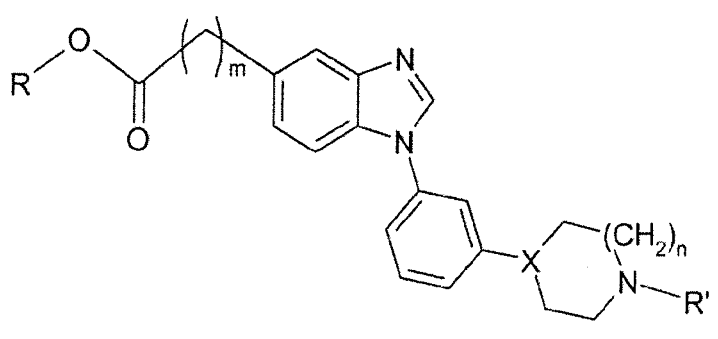

(I)

oder ein N-Oxid davon, oder ein pharmazeutisch annehmbares Salz davon, worin R $C_{1-6}$-Alkyl, Hydroxy-$C_{1-6}$-alkyl, $C_{1-6}$-Alkoxy-$C_{1-6}$-alkyl, $R^aR^bN$-$C_{1-6}$-Alkyl, $R^aR^bN$-CO-$C_{1-6}$-Alkyl oder Phenyl-$C_{1-6}$-alkyl bedeutet,
worin $R^a$ und $R^b$ unabhängig voneinander Wasserstoff oder $C_{1-6}$-Alkyl bedeutet; R' $C_{1-6}$-Alkoxy-$C_{1-6}$-alkyl, $C_{1-6}$-Alkoxy-$C_{2-6}$-alkenyl oder
$C_{1-6}$-Alkoxy-$C_{2-6}$-alkinyl bedeutet;
m 0 oder 1 ist;
n 1 ist;
X N oder CH bedeutet.

**2.** Verbindung nach Anspruch 1, worin X N bedeutet.

**3.** Verbindung nach Anspruch 1, worin X CH bedeutet.

**4.** Verbindung nach einem der Ansprüche 1 bis 3, worin R $C_{1-6}$-Alkyl, Hydroxy-$C_{1-6}$-alkyl oder $C_{1-6}$-Alkoxy-$C_{1-6}$-alkyl bedeutet.

**5.** Verbindung nach einem der Ansprüche 1 bis 3, worin R $R^aR^bN$-$C_{1-6}$-Alkyl, $R^aR^bN$-CO-$C_{1-6}$-Alkyl oder Phenyl-$C_{1-6}$-alkyl bedeutet;
worin $R^a$ und $R^b$ unabhängig voneinander Wasserstoff, Methyl oder Ethyl bedeutet.

**6.** Verbindung nach einem der Ansprüche 1 bis 5, worin R' $C_{1-6}$-Alkoxy-$C_{1-6}$-alkyl bedeutet.

**7.** Verbindung nach Anspruch 1, welche
2-Methoxyethyl-1-(3-(4-(ethoxyethyl)-1-piperazinyl)-phenyl)-benzimidazol-5-carboxylat **(1a);**
2-Hydroxyethyl-1-(3-(4-ethoxyethyl)-1-piperazinyl)-phenyl)-benzimidazol-5-carboxylat **(1b);**
*n*-Butyl-1-(3-(4-(ethoxyethyl)-1-piperazinyl)-phenyl)-benzimidazol-5-carboxylat **(1c);**
*iso*-Butyl-1-(3-(4-(ethoxyethyl)-1-piperazinyl)-phenyl)-benzimidazol-5-carboxylat **(1d);**
2-Methoxyethyl-1-(3-(4-(methoxyethyl)-1-piperazinyl)-phenyl)-benzimidazol-5-carboxylat **(1e);**
2-Hydroxyethyl-1-(3-(4-(methoxyethyl)-1-piperazinyl)-phenyl)-benzimidazol-5-carboxylat **(1f);**
*n*-Butyl-1-(3-(4-(methoxyethyl)-1-piperazinyl)-phenyl)-benzimidazol-5-carboxylat **(1g);**
*iso*-Butyl-1-(3-(4-(methoxyethyl)-1-piperazinyl)-phenyl)-benzimidazol-5-carboxylat **(1h);**
5-(Methoxycarbonylmethyl)-1-(3-(4-(methoxyethyl)-1-piperazinyl)-phenyl)-benzimidazol **(1i);**
5-(2-Hydroxyethoxycarbonylmethyl)-1-(3-(4-(methoxyethyl)-1-piperazinyl)-phenyl)-benzimidazol **(1j);**
2-Methoxyethyl-1-(3-(1-methoxyethyl-4-piperidinyl)-phenyl)-benzimidazol-5-carboxylat **(8d);**
Benzyl-1-(3-(4-(ethoxyethyl)-1-piperazinyl)-phenyl)-benzimidazol-5-carboxylat **(10a);**
Methylcarbamoylmethyl-1-(3-(4-(ethoxyethyl)-1-piperazinyl)-phenyl)-benzimidazol-5-carboxylat **(10b);**
Ethylcarbamoylmethyl-1-(3-(4-(ethoxyethyl)-1-piperazinyl)-phenyl)-benzimidazol-5-carboxylat **(10c);**
2-Dimethylaminoethyl-1-(3-(4-(ethoxyethyl)-1-piperazinyl)-phenyl)-benzimidazol-5-carboxylat **(10d);**
Benzyl-1-(3-(4-(methoxyethyl)-1-piperazinyl)-phenyl)-benzimidazol-5-carboxylat **(10e);**
Methylcarbamoylmethyl-1-(3-(4-(methoxyethyl)-1-piperazinyl)-phenyl)-benzimidazol-5-carboxylat **(10f);**
Ethylcarbamoylmethyl-1-(3-(4-(methoxyethyl)-1-piperazinyl)-phenyl)-benzimidazol-5-carboxylat **(10g);**
2-Dimethylaminoethyl-1-(3-(4-(methoxyethyl)-1-piperazinyl)-phenyl)-benzimidazol-5-carboxylat **(10h)**;
ist, oder ein pharmazeutisch annehmbares Salz davon.

**8.** Pharmazeutische Zusammensetzung, enthaltend eine therapeutisch wirksame Menge von einer Verbindung nach einem der Ansprüche 1 bis 7, oder einem N-Oxid davon, oder einem pharmazeutisch annehmbaren Salz davon, zusammen mit wenigstens einem pharmazeutisch annehmbaren Träger, Excipiens oder Verdünnungsmittel.

**9.** Verwendung von einer Verbindung nach einem der Ansprüche 1 bis 7, oder einem N-Oxid davon, oder einem pharmazeutisch annehmbaren Salz davon, für die Herstellung eines Medikaments für die Behandlung, Verhütung oder Linderung einer Krankheit oder einer Störung oder eines Zustands bzw. Leidens eines Säugers, einschließlich eines Menschen, wobei es sich bei der Krankheit, der Störung oder dem Zustand bzw. Leiden um Fieberkrämpfe oder Status epilepticus handelt.

**10.** Verwendung von einer Verbindung nach einem der Ansprüche 1 bis 7, oder einem N-Oxid davon, oder einem pharmazeutisch annehmbaren Salz davon, für die Herstellung eines Medikaments zum Einleiten einer Anästhesie, Präanästhesie, Muskelrelaxation oder Sedierung.

**Revendications**

**1.** Composé de formule générale I :

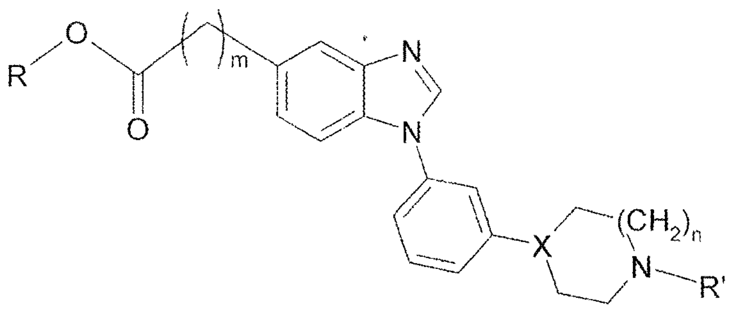

( I )

ou un N-oxyde de celui-ci, ou un sel pharmaceutiquement acceptable de celui-ci, dans laquelle R représente un alkyle en $C_{1-6}$, un hydroxy-alkyle en $C_{1-6}$, un alcoxy en $C_{1-6}$-alkyle en $C_{1-6}$, un $R^aR^bN$-alkyle en $C_{1-6}$, un $R^aR^bN$-CO-alkyle en $C_{1-6}$ ou un phényl-alkyle en $C_{1-6}$ ;
où $R^a$ et $R^b$, indépendamment l'un de l'autre, représentent un hydrogène ou un alkyle en $C_{1-6}$ ;
R' représente un alcoxy en $C_{1-6}$-alkyle en $C_{1-6}$, un alcoxy en $C_{1-6}$-alcényle en $C_{2-6}$ ou un alcoxy en $C_{1-6}$-alcynyle en $C_{2-6}$ ;
m est 0 ou 1 ;
n est 1 ;
X représente N ou CH.

2. Composé selon la revendication 1, dans lequel X représente N.

3. Composé selon la revendication 1, dans lequel X représente CH.

4. Composé selon l'une quelconque des revendications 1 à 3, dans lequel R représente un alkyle en $C_{1-6}$, un hydroxy-alkyle en $C_{1-6}$ ou un alcoxy en $C_{1-6}$-alkyle en $C_{1-6}$.

5. Composé selon l'une quelconque des revendications 1 à 3, dans lequel R représente un $R^aR^bN$-alkyle en $C_{1-6}$, un $R^aR^bN$-CO-alkyle en $C_{1-6}$ ou un phényl-alkyle en $C_{1-6}$ ; où $R^a$ et $R^b$, indépendamment l'un de l'autre, représentent un hydrogène, un méthyle ou éthyle.

6. Composé selon l'une quelconque des revendications 1 à 5, dans lequel R' représente un alcoxy en $C_{1-6}$-alkyle en $C_{1-6}$.

7. Composé selon la revendication 1, qui est
le 1-(3-(4-(éthoxyéthyl)-1-pipérazinyl)-phényl)-benzimidazole-5-carboxylate de 2-méthoxyéthyle **(1a) ;**
le 1-(3-(4-(éthoxyéthyl)-1-pipérazinyl)-phényl)-benzimidazole-5-carboxylate de 2-hydroxyéthyle **(1b) ;**
le 1-(3-(4-(éthoxyéthyl)-1-pipérazinyl)-phényl)-benzimidazole-5-carboxylate de *n*-butyle **(1c) ;**
le 1-(3-(4-(éthoxyéthyl)-1-pipérazinyl)-phényl)-benzimidazole-5-carboxylate d'iso-butyle **(1d) ;**
le 1-(3-(4-(méthoxyéthyl)-1-pipérazinyl)-phényl)-benzimidazole-5-carboxylate de 2-méthoxyéthyle **(1e) ;**
le 1-(3-(4-(méthoxyéthyl)-1-pipérazinyl)-phényl)-benzimidazole-5-carboxylate de 2-hydroxyéthyle **(1f) ;**
le 1-(3-(4-(méthoxyéthyl)-1-pipérazinyl)-phényl)-benzimidazole-5-carboxylate de *n*-butyle **(1g) ;**
le 1-(3-(4-(méthoxyéthyl)-1-pipérazinyl)-phényl)-benzimidazole-5-carboxylate d'*iso*-butyle (1**h**) ;
le 5-(méthoxycarbonylméthyl) 1-(3-(4-(méthoxyéthyl)-1-pipérazinyl)-phényl)-benzimidazole (**1i**) ;
le 5-(2-hydroxyéthoxycarbonylméthyl) 1-(3-(4-(méthoxyéthyl)-1-pipérazinyl)-phényl)-benzimidazole (**1j**) ;
le 1-(3-(1-méthoxyéthyl-4-pipéridinyl)-phényl)-benzimidazole-5-carboxylate de 2-méthoxyéthyle (**8d**) ;
le 1-(3-(4-(éthoxyéthyl)-1-pipérazinyl)-phényl)-benzimidazole-5-carboxylate de benzyle (**10a**) ;
le 1-(3-(4-(éthoxyéthyl)-1-pipérazinyl)-phényl)-benzimidazole-5-carboxylate de méthylcarbamoylméthyle (**10b**) ;
le 1-(3-(4-(éthoxyéthyl)-1-pipérazinyl)-phényl)-benzimidazole-5-carboxylate d'éthylcarbamoylméthyle (**10c**) ;
le 1-(3-(4-(éthoxyéthyl)-1-pipérazinyl)-phényl)-benzimidazole-5-carboxylate de 2-diméthylaminoéthyle (**10d**) ;
le 1-(3-(4-(méthoxyéthyl)-1-pipérazinyl)-phényl)-benzimidazole-5-carboxylate de benzyle (**10e**) ;
le 1-(3-(4-(méthoxyéthyl)-1-pipérazinyl)-phényl)-benzimidazole-5-carboxylate de méthylcarbamoylméthyle (**10f**) ;
le 1-(3-(4-(méthoxyéthyl)-1-pipérazinyl)-phényl)-benzimidazole-5-carboxylate d'éthylcarbamoylméthyle (**10g**) ;
le 1-(3-(4-(méthoxyéthyl)-1-pipérazinyl)-phényl)-benzimidazole-5-carboxylate de 2-diméthylaminoéthyle (**10h**) ;
ou un sel pharmaceutiquement acceptable de celui-ci.

8. Composition pharmaceutique contenant une quantité thérapeutiquement efficace d'un composé selon l'une quelconque des revendications 1 à 7, ou d'un N-oxyde de celui-ci, ou d'un sel pharmaceutiquement acceptable de celui-ci, conjointement avec au moins un véhicule, excipient ou diluant pharmaceutiquement acceptable.

**9.** Utilisation d'un composé selon l'une quelconque des revendications 1 à 7, ou d'un N-oxyde de celui-ci, ou d'un sel pharmaceutiquement acceptable de celui-ci, pour la fabrication d'un médicament pour le traitement, la prévention ou l'atténuation d'une maladie ou d'un trouble ou d'une affection d'un mammifère, incluant un être humain, laquelle maladie, lequel trouble ou laquelle affection est des crampes fébriles ou un état de mal épileptique.

**10.** Utilisation d'un composé selon l'une quelconque des revendications 1 à 7, ou d'un N-oxyde de celui-ci, ou d'un sel pharmaceutiquement acceptable de celui-ci, pour la fabrication d'un médicament pour induire une anesthésie, une pré-anesthésie, une myorelaxation ou une sédation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 616807 A
- WO 9633194 A
- WO 9633191 A
- WO 9633192 A
- WO 9834923 A
- WO 9817651 A
- WO 0078728 A
- WO 02050057 A


### Non-patent literature cited in the description


- **WIKLUND et al.** *The New England Journal of Medicine,* 1997, vol. 337 (16), 1132-1141
- **JAQUES J ; COLLET A ; WIDEN S.** Enantiomers, Racemates, and Resolutions. John Wiley and Sons, 1981
- Remington's Pharmaceutical Sciences. Maack Publishing Co,
- **KONDO et al.** *J. Med. Chem.,* 1989, vol. 32 (3), 679-682